# EUROPÄISCHE PATENTSCHRIFT

(11) **EP 0 799 610 B1**
(45) Veröffentlichungstag und Bekanntmachung des Hinweises auf die Patenterteilung: **11.07.2001**
(21) Anmeldenummer: 97105478.8
(22) Anmeldetag: 02.04.1997
(51) Int. Cl.: A61J 1/10, A61M 1/28

(54) **Anordnung zum Verabreichen einer medizinischen Flüssigkeit**
Device for administering a medical liquid
Dispositif pour administrer une liquide médicale

(30) Priorität: 05.04.1996 DE 19613678
(43) Veröffentlichungstag der Anmeldung: 08.10.1997
(73) Patentinhaber: Fresenius AG, 61350 Bad Homburg v.d.H (DE)
(72) Erfinder: Heilmann, Klaus, 66606 St. Wendel (DE); Jessen, Claus, 66620 Otzenhausen (DE); Friedrich, Peter, 66613 Saarbrücken (DE); Winter, Josef, 66740 Saarlouis (DE)
(74) Vertreter: Luderschmidt, Schüler & Partner GbR

(56) Entgegenhaltungen:
- EP-A- 0 118 114
- WO-A-83/02061
- DE-A- 2 654 725

## Beschreibung

Die Erfindung betrifft eine Anordnung zum Verabreichen einer medizinischen Flüssigkeit mit einem Folienbeutel aus einem verschweißbaren polymeren Material, der eine die medizinische Flüssigkeit enthaltende Kammer aufweist, die von mindestens einer Schweißnaht begrenzt ist und mit einem Überleitgerät, das eine Überführungsleitung aufweist, von der das eine Ende in die Schweißnaht eingeschweißt ist und diese durchsetzt und das andere Ende mit einem Konnektorstück versehen ist und ein Absperrelement aufweist, das das Ausströmen der Flüssigkeit aus der Kammer in die Überführungsleitung verhindert.

Es sind Infusionsbeutel bekannt, die mit einem Anschlußstück versehen sind, um den Beutel mit einem Überleitgerät verbinden zu können, das einen Überführungsschlauch mit einem Absperrelement und einem Anschlußstück an seinem freien Ende aufweist. Ferner finden Infusionsbeutel mit angeschlossenem Überleitgerät Verwendung. Der Vorteil dieser Vorrichtungen zum Verabreichen von medizinischen Flüssigkeiten liegt darin, daß eine Konnektoranordnung zum Anschluß des Überleitgerätes an den Beutel nicht vorhanden ist, was eine Verringerung der Infektionshäufigkeit zur Folge hat. Um eine Verkeimung des Überleitgerätes zu verhindern, ist es bekannt, den Folienbeutel einschließlich des Überleitgerätes in einen separaten Umbeutel einzuschweißen, der tiefgezogen und evakuiert ist. Als nachteilig erweist sich, daß die zusätzliche Verpackung zu erhöhten Herstellungskosten führt. Darüber hinaus muß der Umbeutel nach dem Gebrauch der Vorrichtung entsorgt werden.

Bei der Peritonealdialyse finden zur Verabreichung einer Spülflüssigkeit in die Peritonealhöhle eines Patienten Anordnungen Verwendung, die nicht nur einen flüssigkeitsgefüllten Beutel, sondern auch einen Leerbeutel aufweisen, der die verbrauchte Spülflüssigkeit nach der Peritonealdialyse aufnimmt. Eine derartige Anordnung ist beispielsweise aus der EP-A-0 488 288 bekannt.

DE-A-1 129 258 beschreibt einen Behälter aus flexiblen Kunststoff für medizinische Flüssigkeiten mit einer beutelartigen Hülle für den sterilen und dichten Abschluß eines rohrförmigen Anschlußstücks. Die beutelartige Hülle wird durch flach aufeinanderliegende Verschlußstreifen gebildet, die das vorstehende Ende des Anschlußstücks dicht umschließen. Sowohl die Verschlußstreifen als auch das rohrförmige Anschlußstücks sind mit dem Beutel fest verschweißt.

US-A-54,183,434 beschreibt einen Beutel zur Lagerung von Blutbestandteilen, der mindestens zwei Einlässe aufweist, die durch taschenähnliche Schutzhüllen hermetisch umschlossen sind. Zum Anschluß der Schlauchleitungen können diese Schutzhüllen durch Auseinanderziehen aufgerissen werden.

Die US-A-5,364,384 beschreibt einen in zwei Kammern unterteilten Folienbeutel, wobei die erste Kammer eine medizinische Flüssigkeit aufnimmt und die zweite Kammer die Anschlußstutzen eines Ports steril verpackt umschließt. Die zweite Kammer kann mittels einer Lasche aufgerissen werden, so daß die Anschlußstutzen freiliegen.

Aus der EP-A-0 118 114 ist ein Doppelkammerbeutel für Stoffaustauschapparate zur extrakorporalen Blutreinigung bekannt. Der Beutel besteht aus einer Beutelhülle, die durch eine Trennwand in eine erste Kammer zum Aufnehmen der dem Körperkreislauf entnommenen Flüssigkeit und in eine zweite Kammer für die zurückzuführende Ersatzflüssigkeit unterteilt ist. Das Überleitgerät zum Zuführen der Flüssigkeit ist nicht Bestandteil des Beutels.

Der Erfindung liegt die Aufgabe zugrunde eine einfache und kostengünstig herzustellende Anordnung zum Verabreichen einer medizinischen Flüssigkeit zu schaffen, die vor Verkeimung geschützt ist.

Die Lösung dieser Aufgabe erfolgt erfindungsgemäß mit den Merkmalen des Patentanspruchs 1.

Bei der erfindungsgemäßen Anordnung zum Verabreichen einer medizinschen Flüssigkeit wird die sterile Verpackung durch die Beutelfolie selbst gebildet. Eine zusätzliche Umhüllung kann somit entfallen. Die erfindungsgemäße Anordnungn läßt sich kostengünstig in wenigen Arbeitsschritten herstellen, wobei nur ein Minimum an Verpackungsmaterial erforderlich ist. Die Materialeinsparung führt darüber hinaus zu einem verringerten Gewicht.

Der Folienbeutel weist eine die medizinische Flüssigkeit enthaltende Kammer und eine Kammer auf, die das Überleitgerät aufnimmt, so daß beide Teile dicht umschlossen sind. Um die erfindungsgemäße Anordnung bei der Peritonaldialyse zur Verabreichung einer Spülflüssigkeit in die Peritonealhöhle des Patienten einsetzen zu können, ist eine weitere Kammer vorgesehen, die mit dem freien Ende der von dem Überführungsschlauch des Überleitgerätes abzweigenden Abflußleitung in Fluidverbindung steht. In diese Leerkammer wird die verbrauchte Dialysierflüssigkeit geleitet.

Die flüssigkeitsbefüllte Kammer und die Leerkammer werden durch jeweils zwei übereinanderliegende und miteinander verschweißte Folienlagen gebildet, wobei sich die äußeren Folienlagen über den Randbereich der inneren Folienlagen erstrecken. Die Randbereiche der beiden äußeren Folienlagen sind unter Bildung der das Überleitgerät aufnehmenden Kammer miteinander verschweißt, wobei die inneren Folienlagen flach aufeinanderliegen. In einer alternativen Ausführungsform besteht der Beutel aus drei übereinanderliegenden Folienlagen, wobei die innenliegende Folienlage eine gemeinsame Trennwand für die flüssigkeitsgefüllte Kammer und die Leerkammer bildet.

Um das Überleitgerät entnehmen zu können, muß die dritte Kammer des Folienbeutels geöffnet werden. Die Kammer kann beispielsweise mit einem Messer oder einer Schere ausgeschnitten werden.

Von besonderem Vorteil ist, wenn die umlaufende Schweißnaht, die die äußeren Folienlagen miteinander verbindet, zumindest über einen Teil ihrer Länge peelbar ist, d.h. die aufeinanderliegenden Folien sind entlang der Naht derart miteinander verbunden, daß die Folien ohne Zerstörung trennbar sind. Eine derartige Peelnaht erlaubt es, die Folienlagen des Beutels auf einfache Weise von Hand zu trennen und das Überleitgerät schnell zu entnehmen. Um das Aufreißen des Beutels weiter zu vereinfachen, können noch Aufreißlaschen vorgesehen sein. Alternativ können die Folienlagen aber auch unter Bildung eines schmalen äußeren Randes trennbar miteinander verbunden sein, so daß sich die nicht miteinander verbundenen Folienränder leicht greifen lassen.

Im folgenden wird ein Ausführungsbeispiel der Erfindung unter Bezugnahme auf die Zeichnung näher erläutert.

Es zeigen:
- Fig. 1: eine Anordnung zur Verabreichung einer Spülflüssigkeit in die Peritonealhöhle des Patienten gemäß der Erfindung mit einer flüssigkeitsgefüllten Kammer, einer Leerkammer und einer das Überleitgerät aufnehmenden Kammer in vereinfachter schematischer Darstellung und
- Fig. 2: einen Schnitt durch die Anordnung von Fig. 1 entlang der Linie II-II, wobei die Folienstücke der besseren Übersichtlichkeit wegen nicht maßstabsgetreu dargestellt sind.

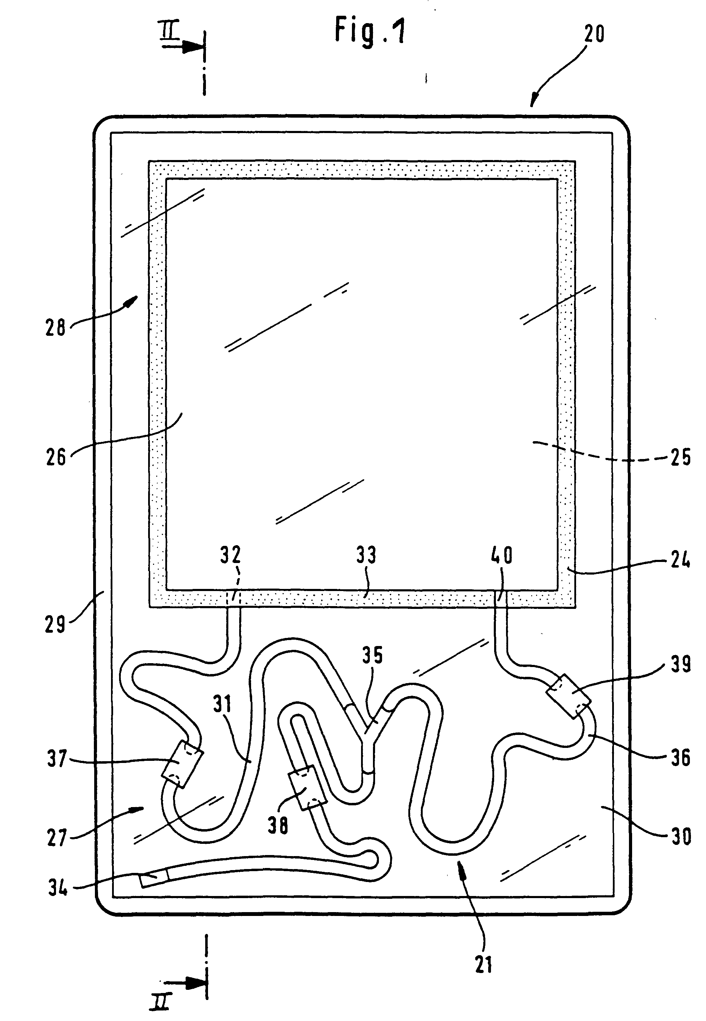

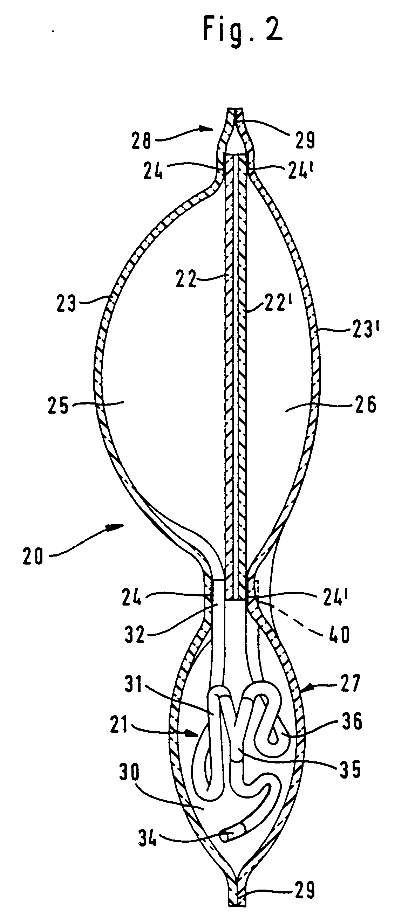

Fig. 1 zeigt ein Ausführungsbeispiel des Folienbeutels 20 mit einem Überleitgerät 21 zur Verabreichung einer Spülflüssigkeit in die Peritonealhöhle eines Patienten in vereinfachter schematischer Darstellung. Der Folienbeutel 20 ist bei diesem Ausführungsbeispiel als Dreikammerbeutel ausgebildet. Er besteht aus vier übereinanderliegenden rechteckförmigen Folienstücken 22, 22' und 23, 23', die unter Ausbildung der drei Kammern miteinander verschweißt sind (Fig. 2). Die Folienstücke 22, 22' und 23, 23' bestehen aus einem nach Verschweißung peelbaren polymeren Material.

Die beiden äußeren Folienstücke 23, 23' sind derart bemessen, daß sich diese über den Randbereich der beiden inneren Folienstücke 22, 22' erstrecken. Die inneren Folienstücke 22 bzw. 22' sind jeweils mit den an diesen anliegenden äußeren Folienstücken 23 bzw. 23' fest verschweißt. Die umlaufenden Schweißnähte in den Außenrandbereichen der inneren Folien 22, 22' sind mit den Bezugszeichen 24, 24' versehen.

Das mit dem Bezugszeichen 23 versehene äußere Folienstück bildet mit dem inneren Folienstück 22 eine Kammer 25, die mit der Spülflüssigkeit gefüllt ist, während das mit dem Bezugszeichen 23' versehene äußere Folienstück mit der inneren Folie 22' eine Leerkammer 26 bildet, die nach der Dialysebehandlung die Spülflüssigkeit aufnimmt.

Die Folienstücke 22, 22' und 23, 23' liegen derart aufeinander, daß sich die äußeren Folienstücke 23, 23' im Bereich der unteren Schmalseite des Beutels in einem breiten Abschnitt 27 überlappen. Ansonsten bilden die überstehenden Randbereiche der äußeren Folienstücke 23, 23' nur einen schmalen Überlappungsbereich 28. Die äußeren Folienstücke 23, 23' sind im Außenrandbereich mit einer peelbaren umlaufenden Schweißnaht 29 verschweißt, so daß eine weitere Kammer 30 gebildet wird, die das in den Fign. 2 und 3 nur andeutungsweise dargestellte Überleitgerät 21 enthält.

Das Überleitgerät 21 weist einen Überführungsschlauch 31 auf, von dem das eine Ende 32 in den unteren Abschnitt 33 der umlaufenden Schweißnaht 24 eingeschweißt ist, die die flüssigkeitsgefüllte Kammer 25 begrenzt, so daß dieser mit dem Kammerinnern in Fluidverbindung steht. An seinem anderen Ende ist der Überführungsschlauch 31 mit einem Anschlußstück 34 versehen, an das ein nicht in der Kammer 30 befindlicher Peritonealkatheter angeschlossen werden kann.

Über ein Y-förmiges Abzweigverbindungsstück 35 ist an den Überführungsschlauch 31 ein Abflußschlauch 36 angeschlossen, dessen freies Ende 33 in den unteren Abschnitt des umlaufenden Schweißrandes 24' eingeschweißt ist, der die leere Kammer 26 begrenzt. In dem Abschnitt des Überführungsschlauchs zwischen der flüssigkeitsgefüllten Kammer 25 und dem Y-förmigen Abzweigverbindungsstück 35 sowie dem Abschnitt zwischen dem Y-förmigen Abzweigverbindungsstück 35 und dem Anschlußstück 34 des Überführungsschlauches 31 ist jeweils ein Absperrelement 37, 38 vorgesehen. Ferner ist ein Absperrelement 39 in der Abflußleitung 36 vorgesehen. Die Absperrelemente 37, 38 und 39 erlauben es, den Überführungsschlauch 31 zu öffnen und den Abflußschlauch 36 zu schließen, so daß die Spülflüssigkeit über das Anschlußstück 34 in den Peritonealkatheter fließen kann bzw. den oberen Abschnitt des Überführungsschlauches 31 zu schließen und den Abflußschlauch 36 zu öffnen, so daß die Spülflüssigkeit aus dem Peritonealkatheter über die Abflußleitung in die leere Kammer geleitet werden kann.

Zum Anschließen des Überleitgerätes 21 an den Peritonealkatheter werden die beiden äußeren Folienstücke 23, 23' entlang der Peelnaht 29 aufgerissen, so daß die flüssigkeitsgefüllte Kammer 25 und die leere Kammer 26 voneinander getrennt werden und das Überleitgerät freiliegt.

## Patentansprüche

1. Anordnung zum Verabreichen einer medizinischen Flüssigkeit mit
einem Folienbeutel (20) aus einem verschweißbaren polymeren Material, der eine erste, die medizinische Flüssigkeit enthaltende Kammer (25) aufweist, die von mindestens einer Schweißnaht (24) begrenzt ist, und
einem Überleitgerät (21), das einen Überführungsschlauch (31) zum Zuführen der medizinschen Flüssigkeit aufweist, von dem das eine Ende (32) in die Schweißnaht (24) eingeschweißt ist und diese durchsetzt und das andere Ende ein Anschlußstück (34) aufweist, wobei
der Beutel (20) vier übereinanderliegende Folienlagen (22, 22', 23, 23') aufweist, von denen die beiden äußeren Folienlagen (23, 23') sich über den Randbereich der inneren Folienlagen (22, 22') erstrecken und die beiden inneren Folienlagen (22, 22') mit den jeweils an diesen anliegenden äußeren Folienlagen (23, 23') unter Bildung der ersten und einer zweiten Kammer (25, 26) verschweißt sind oder der Beutel (21) aus drei übereinanderliegenden Folienlagen zusammengeschweißt ist, wobei die innenliegende Folienlage eine gemeinsame Trennwand für die erste und zweite Kammer (25, 26) bildet,
dadurch gekennzeichnet,
daß die äußeren Folienlagen (23, 23') an ihren Außenrandbereichen unter Bildung einer dritten Kammer (30) miteinander verschweißt sind, die das Überleitgerät (21) steril verpackt enthält, wobei das Überleitgerät eine von dem Überführungsschlauch (31) abzweigende Abflußleitung (36) aufweist, deren freies Ende in die die zweite Kammer (26) begrenzende Schweißnaht (24') eingeschweißt ist.

2. Vorrichtung nach Anspruch 1, dadurch gekennzeichnet, daß die im Außenrandbereich der äußeren Folienlagen (23, 23') verlaufende Schweißnaht (29) zumindest über einen Teil ihrer Länge derart ausgebildet ist, daß die Schweißnaht peelbar ist.

## Claims

1. An arrangement for administering a medicinal fluid, with
a film bag (20) of a weldable polymer material comprising a first chamber (25) containing the medicinal fluid and which is bounded by at least one welded seam (24), and
a transfer device (21) comprising a transfer tube (31) for feeding the medicinal fluid, and one end (32) of which is welded into the welded seam (24) and passes through it, while the other end has a connecting piece (34), whereby the bag (20) comprises four superimposed layers of film (22, 22', 23, 23') of which the two outer film layers (23, 23') extend beyond the marginal portion of the inner film layers (22, 22') while the two inner film layers (22, 22') are welded to the respective outer film layers (23, 23') which bear on them, forming the first and second chambers (25, 26) or the bag (21) is welded together from three superimposed film layers, the inner film layer constituting a common partition for the first and second chambers (25, 26),
characterised in that
the outer film layers (23, 23') are welded to one another at their outer edge portions to form a third chamber (30) which contains the transfer device (21) in sterile packing, the transfer device having, branching from the transfer tube (31), a discharge line (36) the free end of which is welded into the welded seam (24') which bounds the second chamber (26).

2. A device according to claim 1, characterised in that the welded seam (29) extending in the outer marginal portion of the outer film layers (23, 23') is, at least over a part of its length, so constructed that the welded seam is peelable.

## Revendications

1. Dispositif pour administrer un soluté, comprenant
une poche (20), réalisée dans un matériau polymère soudable, la poche comportant un premier compartiment (25) qui contient le soluté et qui est délimité par au moins un cordon de soudure (24), et
un dispositif d'injection (21), qui comporte un tuyau d'injection (31), destiné à acheminer le soluté, et dont l'une des extrémités (32) est soudée dans le cordon de soudure (24) et passe à travers celui-ci, et l'autre extrémité est munie d'un embout de raccordement (34), et
la poche (20) présentant quatre feuilles (22, 22', 23, 23') superposées, parmi lesquelles les deux feuilles extérieures (23, 23') s'étendent au-delà du bord des feuilles intérieures (22, 22') et les deux feuilles intérieures (22, 22') sont soudées chacune aux deux feuilles extérieures (23, 23') respectives en formant le premier et un deuxième compartiment (25, 26) ou la poche (21) est formée par trois feuilles superposées soudées les unes aux autres, la feuille intérieure formant une paroi de séparation commune pour le premier et le deuxième compartiment (25, 26),
caractérisé en ce que
les feuilles extérieures (23, 23') sont soudées l'une contre l'autre sur leur bord extérieur de manière à former un troisième compartiment (30), dans lequel est enveloppé de manière stérile le dispositif d'injection (21), le dispositif d'injection étant muni d'un tuyau d'évacuation (36) monté en dérivation sur le tuyau d'injection (31) et dont l'extrémité libre est soudée dans le cordon de soudure (24') délimitant le deuxième compartiment (26).

2. Dispositif selon la revendication 1, caractérisé en ce que le cordon de soudure (29), qui s'étend sur le bord extérieur des feuilles extérieures (23, 23'), est réalisé au moins sur une partie de sa longueur de telle sorte que le cordon de soudure soit séparable.
